# EUROPEAN PATENT SPECIFICATION

(11) **EP 2 554 652 B1**
(45) Date of publication and mention of the grant of the patent: **05.01.2022**
(21) Application number: 12176950.9
(22) Date of filing: 18.07.2012
(51) Int. Cl.: C12M 1/107

(54) **Anaerobic bioreactor and method for anaerobic conversion of lipid rich biomass to biogas**
Anaerober Bioreaktor und Verfahren zur anaeroben Umwandlung von lipidreicher Biomasse zu Biogas
Bioréacteur anaérobie et procédé de conversion anaérobie d'une biomasse riche en lipides en biogaz

(30) Priority: 01.08.2011 NL 2007207
(43) Date of publication of application: 06.02.2013
(73) Proprietor: Spark Origin B.V., 6511 ZD Nijmegen, (NL)
(72) Inventor: Blankenborg, Stephanus Gerardus Johannes, 6511 ZD Nijmegen (NL)
(74) Representative: van Kooij, Adriaan

(56) References cited:
- EP-A1- 1 900 805
- WO-A1-02/15945
- WO-A1-2009/055793
- WO-A1-2011/076444
- DE-U1-202009 017 017
- GB-A- 2 407 088
- US-A1- 2003 059 927
- US-A1- 2004 154 982

## Description

The present invention relates to an anaerobic bioreactor. More specifically, the present invention relates to an anaerobic bioreactor suitable for anaerobic biogas synthesis from a lipid rich biomass. According to a further aspect, the present invention relates to a method for anaerobic conversion of lipid rich biomass to biogas in a bioreactor. According to yet another aspect the present invention relates to the use of an anaerobic bioreactor.

In an oxygen free environment, micro-organisms can convert organic waste to biogas. Biogas is a mixture of CO2 and CH4 . Further, small amounts of H2 S are formed as well as residue and waste water.

Organic waste, for example food waste, comprises amongst others carbohydrates, such as sugars, as well as proteins and lipids (fats). Carbohydrates can be converted easily in a bioreactor. The conversion of lipids in a bioreactor is problematic as a result of the tendency to form large amounts of foam. This foam is a disadvantage since it inhibits the bacterial conversion and furthermore because it may block the inlets and outlets of the anaerobic bioreactor.

Anaerobic conversion, also known as fermentation or anaerobic digestion, is a common process in the waste-disposal. There are many anaerobic bioreactors for the conversion of manure and waste water.

The anaerobic conversion process is usually divided into the following four sub-processes or phases: Hydrolysis, acidogenesis, acetogenesis and methanogenesis. During these consecutively proceeding phases, larger biodegradable molecules such as carbohydrate polymers, proteins and fats are converted into smaller compounds, which eventually leads to the production of biogas. See also www.wastewaterhandbook.com/webpg/th_sludge_83anaeroc_digesti on.htm.

An anaerobic bioreactor, is a container or vessel usually comprising a stirrer, wherein the content of the container can be maintained at a desired temperature. To the bioreactor waste is added and processed material can be extracted, thereby also providing biogas.

US2003059927 discloses a method and apparatus for anaerobic treatment of organic wastes in a liquid filled vessel wherein particles of the waste are buoyed and form a floating bed in the liquid by the gasses generated by the anaerobic digestion of the wastes. The bed forms to an upper stratum of the least digested, least dense waste and a lower stratum of the most digested, most dense waste. Liquid from beneath the bed is continuously sprayed over the upper surface of the bed through a series of discharge ports which expands to bed downwardly. Each spraying period is followed by a quiescent period to allow the bed to reform. Over time the floating bed is moved from an inlet end to an outlet end of the vessel where the most digested waste is removed. GB2407088 discloses a process and apparatus for treating solid waste anaerobically. Solid waste is collected and transferred to an anaerobic solids digestion vessel into which a liquid containing active bacteria from a tank in a series of anaerobic liquid digester tanks is fed. After anaerobic solids digestion phase, the liquid is drained into the first of the series of anaerobic liquid digester tanks and the treated solids transferred to a de-watering unit to produce de-watered treated solid waste.

WO2011/076444 discloses to a device and method for dry fermenting and/or post-rotting and/or intensive-rotting freely flowing, poorly flowing or non-flowing or pourable processing products, in particular biomass. The device - which optionally can be designed to be gas-tight - consists of a container (1) comprising side walls (2), an upper unit (3) - which optionally can be designed as a gas-tight lock - for feeding the processing product, and a lower unit (4) forming the bottom (5) of the container (1) for discharging the processing product, said lower unit being made of moveable elements, for example a plurality of rotatable rolls (4 a - f) having protrusions (6) that form the bottom or an intermediate bottom (5) of the container (1).

Anaerobic bioreactors suffer from the formation of a floating layer which is also known as scum layer or floating scum layer, which floating layer comprises grease, oils and other floating subject matter. This floating layer is disadvantageous due to several reasons. First of all, the floating layer makes insufficient contact with micro-organisms able to convert the floating layer constituents. Further, the floating layer prevents dissolving of agents. Still further, the floating layer inhibits gas transfer thereby preventing the recovery of produced biogas. Also, the floating layer takes up a lot of limited bioreactor space.

Accordingly, the prior art mainly describes attempts to remove the disadvantageous floating layer. Also processes to minimize the size of the floating layer are known.

Considering the above, there remains a need in the art for a bioreactor suitable for the conversion of lipid rich biomass such as oils, fats and the like, to biogas, which reactor is not hindered by the above mentioned drawbacks.

Therefore, it is an object of the present invention, amongst other objects, to provide an anaerobic bioreactor suitable for anaerobic conversion from a lipid rich biomass to biogas.

More specifically, the present invention relates to an anaerobic bioreactor according to claim 1. Further embodiments are provided in the dependent claims.

During operation of the present anaerobic bioreactor, the lipid rich layer, preferably a solid lipid rich layer, is maintained whereas in prior art bioreactors the lipid rich layer is minimized or removed. Accordingly, the present lipid rich layer substantially remains intact, whereby the conversion, or degradation, of the lipid rich layer may take place via gradual transport of material from the lipid rich layer to the conversion layer and by spraying fluid from the conversion layer on the lipid rich layer. The Lipid rich layer which substantially remains intact as used in the present context means that the present lipid rich layer is not completely or substantially mixed with the present conversion layer, but remains a separate, distinguishable, layer from the conversion layer, although which is in contact with the conversion layer and is situated above the conversion layer. Further, it is preferred when the amount of lipid rich material added to the present anaerobic bioreactor equals the amount of conversed lipid rich layer, therewith maintaining the thickness of the lipid rich layer within a certain range.

Surprisingly, the present inventors found that by maintaining the lipid rich layer, or fat layer, the present anaerobic bioreactor is able to convert lipid rich biomass, such as fats and oils, to biogas, wherein the lipid rich layer provided the gradual and controlled addition of lipid rich material. Further, the present anaerobic bioreactor does not suffer from undesired foaming of the conversion layer. The lipid rich layer floats on the present conversion layer, and therefore can also be designated as a floating layer. Further, the lipid rich layer could also be designated as a scum layer. The lipid rich layer preferably comprises fermentable lipid rich biomass, such as lipid rich food waste, restaurant waste and the like. The lipid rich layer is in contact with the conversion layer, thereby facilitating anaerobic conversion from lipid rich biomass to biogas. Preferably, the lipid rich layer is not mixed with the conversion layer, although small amounts of emulsions of lipid rich biomass in may be present in the conversion layer. The pH of the lipid rich layer is lower than the pH of the conversion layer. Preferably the pH of the lipid rich layer is gradual increasing from the top of the lipid rich layer to the bottom of the lipid rich layer. Preferably, the viscosity of the lipid rich layer is larger than the viscosity of the conversion layer, thereby facilitating maintaining of the lipid rich layer during the conversion from lipid rich biomass to biogas. Preferably, the thickness of the present lipid rich layer is in the range from 20 mm to 200 mm.

The present biogas layer is formed by the anaerobic conversion, or digestion, from the lipid rich biomass. The present outlet on top of the anaerobic bioreactor, or air valve, is configured to remove the present biogas without creating pressure differences which bring the present lipid rich layer in movement. Preferably, the present variation in pressure when biogas is recovered is within the range of 1 to 30 mbar, more preferably in the range of 1 to 20 mbar such as 1 to 5 mbar.

The present anaerobic bioreactor comprises spraying means which are arranged above the level of the lipid rich layer and spray fluid, or liquid, extracted from the conversion layer on top of the lipid rich layer. Thereby, micro-organisms from the conversion layer are introduced on the lipid rich layer, which facilitates anaerobic conversion from lipid rich biomass to biogas. Further, spraying fluid on the lipid rich layer prevents dehydration of the lipid rich layer, thereby reducing the risk of breakage of the lipid rich layer.

The present conversion layer, fermentation layer or digestion layer, is preferably a liquid layer. The conversion layer may comprise supernatant, nutrients, digested biomass, water and/or micro-organisms such as bacteria such as methanobacter, methanosarcina, clostridium, syntrophomonas, yeast and enzymes such as proteases and lipases. Preferably, the pH of the conversion layer is within the range of 6.5 to 7.5. Preferably, the present conversion layer comprises only small amounts of lipid rich material from the lipid rich layer.

The present inlet and outlet for addition or removal of liquid from the conversion layer enables the possibilities to adjust the volume of the conversion layer. further, the outlet situated on the underside, or bottom, of the anaerobic bioreactor for removing precipitated residue enables the removal of waste, generally in solid form, from the conversion layer, without hindering the operation of the anaerobic bioreactor. Thus, it is not necessary to stop the present anaerobic bioreactor.

Preferably, the present outlet configured for removing non fermentable subject matter from the lipid rich layer and/or conversion layer is situated, configured for, or arranged, in the anaerobic bioreactor such that non fermentable subject matter from the lipid rich layer and/or conversion layer can be removed. Non fermentable subject matter comprises constituents which are not susceptible for the anaerobic digestion by the micro-organisms present in the conversion layer. Examples are wood and plastic. The outlet configured for removing non fermentable subject matter from the lipid rich layer and/or conversion layer provides also removal of the entire lipid rich layer if this is desired. This may be advantageous, for example for cleaning the process and anaerobic bioreactor and filling the anaerobic bioreactor with fresh biomass to form a new lipid rich layer. Preferably, the present inlet for lipid rich biomass is configured to introduce lipid rich biomass to the anaerobic bioreactor wherein the present lipid rich layer remains intact.

According to a preferred embodiment, the present anaerobic bioreactor further comprises:
- (vii) a stirrer configured for stirring the conversion layer wherein the lipid rich layer remains intact; and/or
- (viii) heating means configured for heating the conversion layer.

Preferably, the present heating means are configured for heating the conversion layer to a temperature in the range of 5°C to 95°C, preferably of 25°C to 45°C, more preferably in the range of 30°C to 40°C.

According to a preferred embodiment, the present anaerobic bioreactor is **characterized in that** the pH of the lipid rich layer is from 4 to 6.5, preferably from 4.5 to 5.5. More preferably, the pH increases from top to bottom of the present lipid rich layer from 4.5 to 6.5.

According to a preferred embodiment, the present anaerobic bioreactor is **characterized in that** it comprises at least one, preferably two or at least two, pressure sensor configured for measuring the weight of the anaerobic bioreactor.

Given the advantageous properties of the present anaerobic bioreactor for the conversion of lipid rich biomass to biogas, the present invention relates according to a further aspect to a method for anaerobic conversion of lipid rich biomass to biogas in an anaerobic bioreactor comprising the steps of:
- (i) adding the lipid rich biomass to the anaerobic bioreactor thereby forming a lipid rich layer situated above and in contact with a conversion layer for the conversion of the lipid rich biomass to biogas which conversion layer comprises micro-organisms;
- (ii) tapping a fluid, or liquid, comprising micro-organisms from the conversion layer;
- (iii)spraying the fluid, or liquid, tapped from the conversion layer on top of the lipid rich layer wherein the lipid rich layer substantially remains intact; and
- (iv) recovery of the produced biogas;
wherein the lipid rich layer has a pH which is lower than the pH of the conversion layer.

Preferably, in the present method the present anaerobic bioreactor is used.

Further, the present method may comprise a step of adding a liquid to the anaerobic bioreactor for forming a conversion layer.

The present adding of the lipid rich biomass to the anaerobic bioreactor thereby forming a lipid rich layer may be continuously or discontinuously. Preferably, the lipid rich biomass is added to the anaerobic bioreactor for maintaining the present lipid rich layer in a desired thickness, preferably within the range of 20 to 200 mm. More preferably, the lipid rich biomass is added discontinuously, i.e. with time intervals, thereby adding the biomass wherein the lipid rich layer remains intact. It is preferred when the amount of lipid rich material added to the present anaerobic bioreactor equals the amount of digested lipid rich layer, therewith maintaining the thickness of the lipid rich layer within a certain range, preferably in the range of 20 mm to 200 mm.

The present tapping of fluid, or liquid, from the conversion layer is preferably tapping without breakage of the lipid rich layer. Accordingly, by tapping fluid from the conversion layer, the level of the conversion layer, and therewith the level of the lipid rich layer may lower somewhat, but the lipid rich layer remains intact.

The present recovery of the produced biogas is preferably an automatic process which only recovers biogas when a certain buildup in pressure, preferably of 5 to 20 mbar, is perceived by an air valve or outlet for biogas. More preferably, the biogas is recovered without creating a variation in pressure in the anaerobic bioreactor which is larger than 5, 10, 15 or 20 mbar.

Preferably, the present spraying of the fluid tapped from the conversion layer on top of the lipid rich layer occurs at least once a day.

According to a preferred embodiment, the present method comprises:
- v) heating the conversion layer to a temperature in the range of 5°C to 95°C, preferably of 25°C to 45°C, more preferably in the range of 30°C to 40°C.

According to a preferred embodiment, the present method comprises:
- (vi) continue or discontinue stirring of the conversion layer wherein the lipid rich layer remains intact.
Preferably, the present conversion layer is stirred each hour. More preferably, the present conversion layer is stirred at least two times per hour. The revs per minute of the stirrer are preferably within the range of 10 to 50 revs per minute (rpm). Stirring of the conversion layer is advantageous for conversion of the lipid rich layer.

According to a preferred embodiment, the present method comprises:
- (vi) adding or tapping fluid, or liquid, to or from the conversion layer thereby adjusting the volume of the conversion layer, preferably wherein the lipid rich layer substantially remains intact.
Preferably, the present bioreactor is not stirred for a period of time enough to separate the conversion layer in a thicker fraction, or more viscous fraction, and a thinner, liquid, fraction. Preferably, the thick fraction comprises bacteria. Preferably, the liquid fraction comprises waste water. More preferably, tapping from the fluid from the conversion layer (step vi) comprises tapping said liquid fraction from the conversion layer, wherein the thick fraction comprising bacteria substantially remains in the anaerobic bioreactor.

According to a preferred embodiment, the present method comprises:
- (vii)weighing the mass of the anaerobic bioreactor by at least one pressure sensor.

Preferably, when the at least one pressure sensor perceives a decline in volume of the present anaerobic bioreactor, than fluid, or liquid, will automatically added to the anaerobic bioreactor, thereby bringing the volume of the conversion layer to a desired volume.

According to a preferred embodiment, the present method comprises:
- (ix) removing non fermentable subject matter from the lipid rich layer and/or conversion layer.
The advantage of removing the present non fermentable subject matter, or removing a part or the entire lipid rich layer, is that the anaerobic bioreactor can be cleaned efficiently.

According to yet another preferred embodiment, the present method comprises:
- (x) removing precipitated residue from the underside of the anaerobic bioreactor.
The advantage of removing precipitated residue is that the reactor volume of the anaerobic bioreactor is not wasted to precipitated residue, which enhances the efficacy of the anaerobic bioreactor.

According to a further aspect, the present invention relates to the use of the present anaerobic bioreactor for the conversion of lipid rich biomass to biogas.

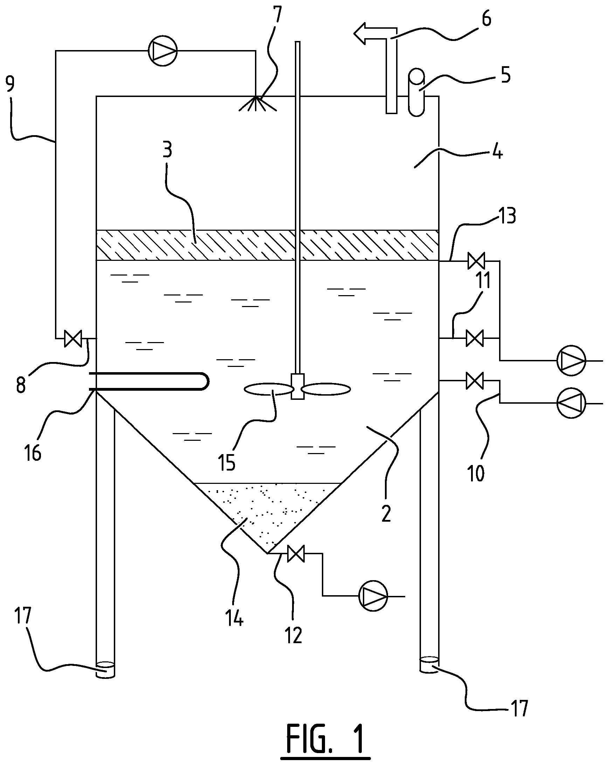

The principles of the present invention will be further detailed in the example showing a preferred embodiment of the present invention. In the example reference is made to figure 1 , showing a schematic view of an anaerobic bioreactor according to the present invention.

### Example Anaerobic bioreactor

In figure 1 is shown an anaerobic bioreactor (1) comprising a conversion layer (2) for anaerobic conversion from lipid rich biomass to biogas. Further, the anaerobic bioreactor comprises a lipid rich layer (3), a biogas layer (4), and inlet for the lipid rich biomass (5). The inlet (5) is used to add lipid rich biomass to the lipid rich layer (3), wherein the lipid rich layer (3) does not mix with the conversion layer (2). Produced biogas which is present in biogas layer (4) is recovered via the outlet (6), however, wherein the lipid rich layer (3) does not substantially mix with the conversion layer (2). Further is shown the spraying means (7) for spraying liquid from the conversion layer (2) via outlet (8) and conduit (9). Further, the shown anaerobic bioreactor comprises an inlet (10) for adding liquid to the conversion layer (2), an outlet (11) for removing liquid from the conversion layer (2), an outlet (12) for removing precipitated residue (14) and an outlet (13) for removing floating non fermentable subject matter and/or the lipid rich layer (3). Further is shown a stirrer (15) for stirring the conversion layer (2) without mixing the conversion layer (2) with lipid rich layer (3). Also is shown heating means (16) for heating the conversion layer (2). Further are shown pressure sensors (17) for weighing the mass of the anaerobic bioreactor.

### Process

The anaerobic bioreactor (1) having a volume of 5000 dm3 is filled with water comprising bacteria until a volume of 2500 dm3 is reached, thereby forming the conversion layer (2). Each 2 hours, 8 kg lipid rich biomass obtained from restaurants is added via the inlet (5), which lipid rich biomass is supplied via a conduit (not shown) and a pump having a capacity of 240 dm3 per hour. Hereby, the lipid rich layer (3) is formed controlled and gradually.

The conversion layer (2) is heated until a temperature of 38.8 °C is reached and subsequently the heating is stopped. When the temperature of the conversion layer (2) is lowered to 37.6 °C, the heating started again.

Filling of the anaerobic reactor (1) with liquid via inlet (10) and/or inlet (5) is determined by the pressure sensors (17).

The stirrer (15) stirs 1 minute per 3 minutes, at 25 rpm.

When the bioreactor (1) reaches a volume of 3000 dm3 , the heating and stirring cycles stop for 2 hours, thereby generating a separation of the conversion layer (2) into a bacteria rich layer and a waste water layer. Subsequently, the waste water layer is removed from the conversion layer (2) via the outlet (11) until a volume of 2500 dm3 was obtained.

Spraying means (7) sprayed fluid taken from the conversion layer (2) two times per 24 hours for 120 seconds, with a capacity of 50 liter per minute.

For each 20 rounds from 2500 dm3 to 3000 dm3 , 100 dm3 precipitated matter (14) is removed from the anaerobic bioreactor via the outlet (12).

Each 6 months, outlet (13) removed the lipid rich layer (3).

The outlet (6) for the produced biogas (4) removes biogas automatically when the variation in pressure exceeds 5 mbar, until the pressure is lowered with 5 mbar.

### Results

By operating the anaerobic bioreactor (1) according to the above parameters, the lipid rich layer 3 remained within the range of 20 to 200 mm, so the amount of lipid rich material added to anaerobic bioreactor was equal to the amount of lipid rich biomass digested to biogas. Further, by operating the anaerobic bioreactor (1) during 12 months, 40,000 kg of lipid rich biomass was digested, thereby producing 6,000 m3 biogas.

The biogas was of good quality, and could be used as a fuel. Thus, the above anaerobic bioreactor according to the present invention was provides the anaerobic conversion of lipid rich biomass to biogas.

## Claims

1. Anaerobic bioreactor (1) suitable for anaerobic conversion from a lipid rich biomass to biogas, which anaerobic reactor is arranged to comprise during the anaerobic conversion from the lipid rich biomass to biogas the following layers:
(1) a conversion layer (2) for anaerobic conversion from the lipid rich biomass to biogas comprising micro-organisms;
(2) a lipid rich layer (3) having a pH which is lower than the pH of the conversion layer, wherein the lipid rich layer is arranged above the conversion layer and wherein the lipid rich layer is in contact with the conversion layer;
(3) a biogas layer (4) arranged above the lipid rich layer; which anaerobic bioreactor comprises:
(i) an inlet (5) suitable for the lipid rich biomass;
(ii) an outlet (6) on top of the anaerobic reactor suitable for the biogas formed by the anaerobic conversion from the lipid rich layer to biogas;
(iii) an inlet (10) for a fluid suitably arranged for forming a conversion layer;
(iv) an outlet (8) for a fluid suitably arranged for tapping fluid from the conversion layer;
(v) an outlet (12) arranged on the bottom of the anaerobic bioreactor suitable for removing precipitated residue; and
(vi) an outlet (13) suitably arranged for removing non fermentable subject matter from the lipid rich layer and/or conversion layer; wherein the reactor further comprises spraying means (7) arranged above the lipid rich layer, which spraying means are configured to spray continuously or discontinuously a fluid comprising micro-organisms taken from the conversion layer onto the top of the lipid rich layer, such that the lipid rich layer substantially remains intact, wherein the inlet for lipid rich biomass is arranged above the lipid rich layer such that during the anaerobic conversion from the lipid rich biomass to biogas the added lipid rich biomass is poured continuously or discontinuously on the lipid rich layer.

2. Anaerobic bioreactor according to claim 1, **characterized in that** it comprises:
(vii) a stirrer (15) configured for stirring the conversion layer wherein the lipid rich layer substantially remains intact; and/or
(viii) heating means configured for heating the conversion layer.

3. Anaerobic bioreactor according to claim 1 or 2, **characterized in that** it comprises at least one pressure sensor (17) configured for measuring the weight of the anaerobic bioreactor.

4. Method for anaerobic conversion of lipid rich biomass to biogas in an anaerobic bioreactor (1) according to claims1-3 comprising the steps of:
(i) adding the lipid rich biomass to the anaerobic bioreactor thereby forming a lipid rich layer (3) situated above and in contact with a conversion layer (2) for the conversion of the lipid rich biomass to biogas which conversion layer comprises micro-organisms;
(ii) tapping a fluid comprising micro-organisms from the conversion layer;
(iii) spraying the fluid tapped from the conversion layer on top of the lipid rich layer wherein the lipid rich layer substantially remains intact; and
(iv) recovery of the produced biogas; wherein the lipid rich layer has a pH which is lower than the pH of the conversion layer.

5. Method according to claim 4, further comprising:
(v) heating the conversion layer to a temperature in the range of 5°C to 95°C, preferably of 25°C to 45°C, more preferably in the range of 30°C to 40°C.

6. Method according to claim 4 or claim 5, further comprising:
(vi) continuously or discontinuously stirring of the conversion layer wherein the lipid rich layer substantially remains intact.

7. Method according to any of the claims 4-6, further comprising:
(vii) adding or tapping fluid to or from the conversion layer thereby adjusting the volume of the conversion layer.

8. Method according to any of the claims 4-7, further comprising:
(viii) weighing the mass of the anaerobic bioreactor by pressure sensors.

9. Method according to any of the claims 4-8, further comprising:
(ix) removing non-fermentable subject matter from the lipid rich layer and/or conversion layer.

10. Method according to any of the claims 4-9, further comprising:
(x) removing precipitated residue from the bottom of the anaerobic bioreactor.

11. Method according to any of the claims 4-10, wherein the anaerobic bioreactor according to any of the claims 1-3 is used.

## Patentansprüche

1. Anaerober Bioreaktor (1), der für die anaerobe Umwandlung einer lipidreichen Biomasse in Biogas geeignet ist, wobei der anaerobe Bioreaktor derart eingerichtet ist, dass er während der anaeroben Umwandlung der lipidreichen Biomasse in Biogas die folgenden Schichten aufweist:
(1) eine Umwandlungsschicht (2) für die anaerobe Umwandlung der lipidreichen Biomasse in Biogas, die Mikroorganismen aufweist;
(2) eine lipidreiche Schicht (3) mit einem pH-Wert, der niedriger als der pH-Wert der Umwandlungsschicht ist, wobei die lipidreiche Schicht oberhalb der Umwandlungsschicht eingerichtet ist und wobei die lipidreiche Schicht in Kontakt mit der Umwandlungsschicht ist;
(3) eine Biogasschicht (4), die oberhalb der lipidreichen Schicht eingerichtet ist; wobei der anaerobe Bioreaktor aufweist:
(i) einen Einlass (5), der für die lipidreiche Biomasse geeignet ist;
(ii) einen Auslass (6) oben auf dem anaeroben Reaktor, der für das Biogas geeignet ist, das durch die anaerobe Umwandlung der lipidreichen Schicht in Biogas gebildet wird;
(iii) einen Einlass (10) für ein Fluid, der geeignet eingerichtet ist, um eine Umwandlungsschicht zu bilden;
(iv) einen Auslass (8) für ein Fluid, der geeignet eingerichtet ist, um Fluid von der Umwandlungsschicht abzuzapfen;
(v) einen Auslass (12), der auf dem Boden des anaeroben Bioreaktors eingerichtet ist, der geeignet ist, um ausgefällte Rückstände zu entfernen; und
(vi) einen Auslass (13), der geeignet eingerichtet ist, um nicht fermentierbare Stoffe aus der lipidreichen Schicht und/oder Umwandlungsschicht zu entfernen;
wobei der Reaktor ferner Sprüheinrichtungen (7) aufweist, die oberhalb der lipidreichen Schicht eingerichtet sind, wobei diese Sprüheinrichtungen konfiguriert sind, um kontinuierlich oder diskontinuierlich ein Fluid, das Mikroorganismen enthält, das aus der Umwandlungsschicht genommen ist, auf die Oberseite der fluidreichen Schicht zu sprühen, so dass die lipidreiche Schicht im Wesentlichen intakt bleibt, wobei der Einlass für lipidreiche Biomasse oberhalb der lipidreichen Schicht eingerichtet ist, so dass während der anaeroben Umwandlung der lipidreichen Biomasse in Biogas die zugesetzte lipidreiche Biomasse kontinuierlich oder diskontinuierlich auf die lipidreiche Schicht gegossen wird.

2. Anaerober Bioreaktor nach Anspruch 1, **dadurch gekennzeichnet, dass** er aufweist:
(vii) einen Rührer (15), der konfiguriert ist, um die Umwandlungsschicht zu rühren, wobei die lipidreiche Schicht im Wesentlichen intakt bleibt; und/oder
(viii) Heizeinrichtungen, die konfiguriert sind, um die Umwandlungsschicht zu heizen.

3. Anaerober Bioreaktor nach Anspruch 1 oder 2, **dadurch gekennzeichnet, dass** er wenigstens einen Drucksensor (17) aufweist, der konfiguriert ist, um das Gewicht des anaeroben Bioreaktors zu messen.

4. Verfahren für die anaerobe Umwandlung lipidreicher Biomasse in Biogas in einem anaeroben Bioreaktor (1) nach den Ansprüchen 1 - 3, das die folgenden Schritte aufweist:
(i) Zusetzen der lipidreichen Biomasse zu dem anaeroben Bioreaktor, wodurch eine lipidreiche Schicht (3) gebildet wird, die oberhalb von und in Kontakt mit einer Umwandlungsschicht (2) für die Umwandlung der lipidreichen Biomasse in Biogas gelegen ist, wobei die Umwandlungsschicht Mikroorganismen aufweist;
(ii) Abzapfen eines Fluids, das Mikroorganismen aufweist, aus der Umwandlungsschicht;
(iii) Sprühen des aus der Umwandlungsschicht abgezapften Fluids auf die Oberseite der lipidreichen Schicht, wobei die lipidreiche Schicht im Wesentlichen intakt bleibt; und
(iv) Gewinnen des erzeugten Biogases;
wobei die lipidreiche Schicht einen pH-Wert hat, der niedriger als der pH-Wert der Umwandlungsschicht ist.

5. Verfahren nach Anspruch 4, das ferner aufweist:
(v) Heizen der Umwandlungsschicht auf eine Temperatur im Bereich von 5°C bis 95°C, vorzugsweise auf 25°C bis 45°C, bevorzugter auf den Bereich von 30°C bis 40°C.

6. Verfahren nach Anspruch 4 oder Anspruch 5, das ferner aufweist:
(vi) kontinuierliches oder diskontinuierliches Rühren der Umwandlungsschicht, wobei die lipidreiche Schicht im Wesentlichen intakt bleibt.

7. Verfahren nach einem der Ansprüche 4 - 6, das ferner aufweist:
(vii) Zusetzen von Fluid zu der Umwandlungsschicht oder Abzapfen von Fluid daraus, wodurch das Volumen der Umwandlungsschicht eingestellt wird.

8. Verfahren nach einem der Ansprüche 4 - 7, das ferner aufweist:
(viii) Wiegen der Masse des anaeroben Bioreaktors durch Drucksensoren.

9. Verfahren nach einem de Ansprüche 4 -8, das ferner aufweist:
(ix) Entfernen der nicht fermentierbaren Stoffe aus der lipidreichen Schicht und/oder der Umwandlungsschicht.

10. Verfahren nach einem der Ansprüche 4 - 9, das ferner aufweist:
(x) Entfernen von ausgefällten Rückständen von dem Boden des anaeroben Bioreaktors.

11. Verfahren nach einem der Ansprüche 4 - 10, wobei der anaerobe Bioreaktor nach einem der Ansprüche 1 - 3 verwendet wird.

## Revendications

1. Bioréacteur anaérobie (1) approprié pour la conversion anaérobie d'une biomasse riche en lipides en biogaz, lequel réacteur anaérobie est agencé pour comprendre lors de la conversion anaérobie de la biomasse riche en lipides en biogaz les couches suivantes :
(1) une couche de conversion (2) pour la conversion anaérobie de la biomasse riche en lipides en biogaz comprenant des micro-organismes ;
(2) une couche riche en lipides (3) ayant un pH qui est inférieur au pH de la couche de conversion, dans lequel la couche riche en lipides est agencée au-dessus de la couche de conversion et dans lequel la couche riche en lipides est en contact avec la couche de conversion ;
(3) une couche de biogaz (4) agencée au-dessus de la couche riche en lipides ;
lequel bioréacteur anaérobie comprend :
(i) une entrée (5) appropriée pour la biomasse riche en lipides ;
(ii) une sortie (6) au-dessus du réacteur anaérobie appropriée pour le biogaz formé par la conversion anaérobie de la couche riche en lipides en biogaz ;
(iii) une entrée (10) pour un fluide agencée de manière appropriée pour former une couche de conversion ;
(iv) une sortie (8) pour un fluide agencée de manière appropriée pour prélever un fluide à partir de la couche de conversion ;
(v) une sortie (12) agencée au fond du bioréacteur anaérobie appropriée pour éliminer les résidus précipités ; et
(vi) une sortie (13) agencée de manière appropriée pour éliminer la matière non fermentescible à partir de la couche riche en lipides et/ou de la couche de conversion ;
dans lequel le réacteur comprend en outre des moyens de pulvérisation (7) agencés au-dessus de la couche riche en lipides, lesquels moyens de pulvérisation sont configurés pour pulvériser de manière continue ou discontinue un fluide comprenant des micro-organismes prélevés dans la couche de conversion sur le dessus de la couche riche en lipides, de sorte que la couche riche en lipides reste sensiblement intacte, dans lequel l'entrée pour la biomasse riche en lipides est agencée au-dessus de la couche riche en lipides de sorte que lors la conversion anaérobie de la biomasse riche en lipides en biogaz, la biomasse riche en lipides ajoutée est versée de manière continue ou discontinue sur la couche riche en lipides.

2. Bioréacteur anaérobie selon la revendication 1, **caractérisé en ce qu'**il comprend :
(vii) un agitateur (15) configuré pour agiter la couche de conversion, dans lequel la couche riche en lipides reste sensiblement intacte ; et/ou
(viii) des moyens de chauffage configurés pour chauffer la couche de conversion.

3. Bioréacteur anaérobie selon la revendication 1 ou 2, **caractérisé en ce qu'**il comprend au moins un capteur de pression (17) configuré pour mesurer le poids du bioréacteur anaérobie.

4. Procédé de conversion anaérobie d'une biomasse riche en lipides en biogaz dans un bioréacteur anaérobie (1) selon les revendications 1 à 3 comprenant les étapes consistant à :
(i) ajouter la biomasse riche en lipides au bioréacteur anaérobie pour ainsi former une couche riche en lipides (3) située au-dessus et au contact d'une couche de conversion (2) pour la conversion de la biomasse riche en lipides en biogaz, laquelle couche de conversion comprend des micro-organismes ;
(ii) prélever un fluide comprenant des micro-organismes dans la couche de conversion ;
(iii) pulvériser le fluide prélevé dans la couche de conversion sur le dessus de la couche riche en lipides, dans lequel la couche riche en lipides reste sensiblement intacte ; et
(iv) récupérer le biogaz produit ;
dans lequel la couche riche en lipides présente un pH qui est inférieur au pH de la couche de conversion.

5. Procédé selon la revendication 4, comprenant en outre :
(v) le chauffage de la couche de conversion à une température dans la plage de 5 °C à 95 °C, de préférence de 25 °C à 45 °C, de manière davantage préférée dans la plage de 30 °C à 40 °C.

6. Procédé selon la revendication 4 ou la revendication 5, comprenant en outre :
(vi) l'agitation continue ou discontinue de la couche de conversion, dans lequel la couche riche en lipides reste sensiblement intacte.

7. Procédé selon l'une quelconque des revendications 4 à 6, comprenant en outre :
(vii) l'ajout ou le prélèvement d'un fluide à ou à partir de la couche de conversion, pour ainsi ajuster le volume de la couche de conversion.

8. Procédé selon l'une quelconque des revendications 4 à 7, comprenant en outre :
(viii) la pesée de la masse du bioréacteur anaérobie par des capteurs de pression.

9. Procédé selon l'une quelconque des revendications 4 à 8, comprenant en outre :
(ix) l'élimination de la matière non fermentescible à partir de la couche riche en lipides et/ou de la couche de conversion.

10. Procédé selon l'une quelconque des revendications 4 à 9, comprenant en outre :
(x) l'élimination des résidus précipités par le fond du bioréacteur anaérobie.

11. Procédé selon l'une quelconque des revendications 4 à 10, dans lequel le bioréacteur anaérobie selon l'une quelconque des revendications 1 à 3 est utilisé.
